# EUROPEAN PATENT APPLICATION

(11) **EP 2 705 813 A1**
(43) Date of publication of application: **12.03.2014**
(21) Application number: 12183143.2
(22) Date of filing: 05.09.2012
(51) Int. Cl.: A61F 9/008

(54) **Laser configuration that reduces the laser footprint and improves ergonomics**

(71) Applicant: Technolas Perfect Vision GmbH, 80992 München (DE)
(72) Inventor: Paskuloff, Christian, 83624 Otterfing (DE); Seidl, Franz, 82031 Grünwald (DE); Mosedale, Gwillem, 80995 München (DE); Hohla, Martin, 80802 München (DE)
(74) Representative: Vossius & Partner

(57) **Abstract**

A laser system for eye surgery comprises a laser source for emitting a laser beam and a beam delivery means to direct the laser beam to a position where a patient's eye is located. The beam delivery means within a beam delivery arm is arranged at an angle α with respect to the direction of the beam when emitted from the laser source. The angle α is in a range of 105° to 165°, preferably in a range of 115° to 155°, and more preferably the angle α is approximately 135°. The system may comprise a device body in which the laser source may be arranged. The beam delivery means may comprise a beam delivery arm extending to a position above the patient's eye. Preferably, the laser source is arranged to emit the laser beam in a direction from the head to the feet of the patient when being positioned for surgery. That is, the laser beam is emitted in a direction away from an operator of the device.

## Description

The present invention relates to laser systems for eye surgery, and in particular to laser systems having a configuration with reduced footprint and improved ergonomics.

In eye surgery, ophthalmic laser sources are used to output a laser beam in a particular direction relative to a surgically positioned patient. Excimer laser sources are frequently used as ophthalmic laser sources. Typically, upon exiting the laser source, Excimer laser beams travel in a direction that is substantially 'feet-to-head', where 'feet-to-head' refers to the anatomical positions of a patient positioned for surgery. In other words, the laser beam travels towards the head of the patient.

For example, this is the case in the Excimer laser system which is described in EP 0 906 073 A1. In this document, a compact Excimer laser system is disclosed that includes argon fluoride laser gas, electronics, and laser head all compactly arranged such that the patient's bed can rotate over all of these components.

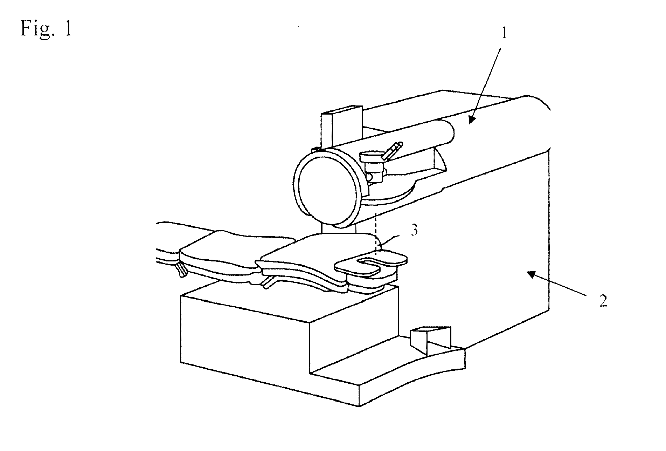

A typical design feature of existing lasers lies in a laser arm which delivers the laser beam vertically from a position above the patient's head. As exemplarily shown in Fig. 1, the laser arm 1 is connected to the device casing 2 at a 90° angle. With the laser arm 1, the laser beam is guided to the position 3 where the patient's eye to be treated is located when the patent is positioned for surgery.

However, the casing of the laser arm usually used in known laser systems makes it hard or impossible for a bystander standing next to the surgeon operating the device to see the patients head. Furthermore, there is still a need in the art to provide a space-saving laser system which may be installed in incommodius treatment rooms.

Accordingly, it is an object of the present invention to provide a laser configuration that reduces the laser footprint and improves ergonomics.

In order to achieve the above object, the present invention provides a laser system for eye surgery comprising a laser source for emitting a laser beam and a beam delivery means to direct the laser beam to a position where a patient's eye is located. Within a beam delivery arm, the beam delivery means is arranged at an angle α with respect to the direction of the beam when emitted from the laser source. The angle α is in a range of 105° to 165°, preferably in a range of 115° to 155°, 125° to 145° or 130° to 140°. More preferably, the angle α is approximately 135°.

The system may comprise a device body in which the laser source may be arranged. The beam delivery arm extends to a position above the patient's eye. Preferably, the beam delivery arm is either fixed on the device body and/or moveable during operation of the laser. The beam delivery arm may further be pivotable with respect to the device body in a horizontal or other plane, e.g. for transport. The beam delivery arm may be mounted to the device body at a position substantially posterior to the position of the shoulders of the patient when being positioned for surgery in an operational position. The beam delivery arm may extend to the position above the patient's eye along a straight line or extend from the laser device in a curved shape.

Preferably, the laser source is arranged to emit the laser beam in a direction from the head to the feet of the patient when being positioned for surgery. That is, the laser beam is emitted in a direction away from an operator of the device.

The laser system, and specifically the device body, may be arranged adjacent to a longitudinal side of a patient bed, chair or table. In this case, the laser beam preferably exits the beam delivery means substantially perpendicular to the plane of the bed, chair or table, the beam axis being adjustable relative to the eye of the patient when positioned for surgery.

The laser device further may further comprise a control unit for controlling the laser source, the beam delivery arm and/or the beam directing means. Furthermore, a display screen may be positioned on top of the device body or the beam delivery arm. Preferably, the control unit and/or the display screen are arranged at a position substantially parallel to the head of the patient when being positioned for surgery.

A laser source that may advantageously be used in the laser system according to the present invention is an Excimer laser, in particular an argon fluoride laser providing a laser beam of approximately 193 nanometers. Alternatively, a solid state UV laser may be used. The laser beam emitted by the laser source may be a pulsed laser beam, with pulse durations of several femtoseconds to several picoseconds.

The system preferably comprises a casing housing the laser source. Preferably, the laser source is a compact laser source. Accordingly, the casing should preferably have a length not greater than 100 cm, a width of at most 35 cm, and/or a height of at most 85 cm or different combinations of values that yield an equivalent volume. It is further preferred that the ratio of the length of the beam delivery arm and the length of the casing is greater than 0.7, more preferably greater than 0.9 and most preferably greater than 1.0.

With the invention a laser configuration is provided that reduces the laser footprint and improves ergonomies. The invention comprises a laser source that is 'reversed' when compared with existing laser orientations. Upon exiting the source, the beam is substantially oriented in a 'head-to-feet direction'. This has the advantage of bringing the attachment of the arm with the main body back or away from the patient's head.

In contrast to existing systems, the attachment point of the beam delivery arm to the device body can be located parallel to where the patient's shoulder is, whereas conventionally is it parallel to the patient's head. This is relevant because the patient head is the focus of attention of both the surgeon and of support staff assisting the surgeon, and it therefore should be easily visible by all. The present invention thus overcomes the drawback of conventional systems where the beam delivery arm is likely to obstruct the view. Alternatively, the attachment point may be further back (posterior) than the patients' shoulders.

Other aspects, features, and advantages will be apparent from the summary above, as well as from the description that follows, including the figures and the claims.

In the following, the present invention is described in more detail with reference to the Figures, wherein
Fig. 1schematically shows a conventional laser system configuration and
Fig. 2 illustrates the configuration of the laser system according to an embodiment of the present invention.

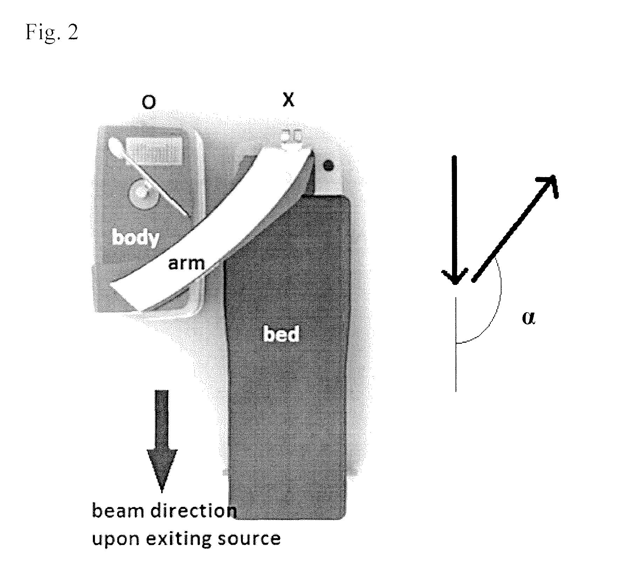

A laser system according to an embodiment of the present invention is shown in Fig. 2. The laser system comprises a body and an arm. The arm is attached to the device body and extends from the device body to the position above the patient's eye in a curved shape. A patient bed is arranged next to the device body so that a patient arranged on the bed in a surgical position can be treated with the laser system.

A laser source is arranged inside the device body. In Fig. 2, the direction of the laser beam upon exiting the laser source is illustrated. As can be seen in Fig. 2, the beam travels in a direction which is parallel to a "head-to-feet" direction of a patient lying on the patient bed. This direction is reversed when compared with the laser source orientation adopted in conventional ophthalmic laser systems.

Using beam delivery means, the laser beam is directed to enter the beam delivery arm and travels along this arm. That is, the direction of the laser beam inside the beam delivery arm has an angle of about 135° compared to the beam direction when exiting the laser source.

This arm configuration at a 135° angle thus constitutes a partial reversal of the beam trajectory when compared with a conventional configuration with a 90° angle given that one of the two vectors describing the arms orientation runs parallel to the patient in a "feet-to-head" direction.

In other words, the configuration according to the present invention may be called a "twice reversed" laser: first, the beam travels in a "head-to-feet" direction upon exiting the laser source, which is counter to the beam direction of existing systems. This may be regarded as the first reversal. Then, the beam enters the arm and a vector component of its trajectory along the arm runs in a "feet-to-head" direction. This component is reversed compared to the beam's initial "head-to-feet" direction, which could be regarded as the second reversal.

The direction of the beam in the beam delivery arm may be described using an angle α which is illustrated in the right portion of Fig. 2. According to this definition, angle α is about 135° in the configuration shown in Fig. 2. According to the present invention, angle α is generally an obtuse angle in the range of 105°-165°. Preferably, angle α is in the range of 115°-155°, 125°-145°, or 130°-140°. More preferably, the angle α is approximately 135°.

During surgery, the surgeon is usually located at the position which is marked with X in Fig. 2. An observer, who may be located at the position O benefits from a markedly improved view of the patient's eye and head without sacrificing access to the laser controls. This is achieved by arranging the beam delivery arm in a position which does not obstruct the view of the observer. In addition, both persons have a clear view of a display screen that may be located on top of the laser's main body.

While the invention has been illustrated and described in detail in the drawings and foregoing description, such illustration and description are to be considered illustrative or exemplary and not restrictive. It will be understood that changes and modifications may be made by those of ordinary skill within the scope of the following claims. In particular, the present invention covers further embodiments with any combination of features from different embodiments described above and below.

Furthermore, in the claims the word "comprising" does not exclude other elements or steps, and the indefinite article "a" or "an" does not exclude a plurality. A single unit may fulfil the functions of several features recited in the claims. The terms "essentially", "about", "approximately" and the like in connection with an attribute or a value particularly also define exactly the attribute or exactly the value, respectively. Any reference signs in the claims should not be construed as limiting the scope.

## Claims

1. A laser system for eye surgery comprising:
a laser source for emitting a laser beam in a beam direction;
a beam delivery means within a beam delivery arm being arranged at an angle α with respect to the beam direction, wherein the angle α is in a range of 105° to 165°, preferably in a range of 115° to 155°, and more preferably the angle α is approximately 135°,
wherein the beam delivery means is configured to direct the laser beam to a position where a patient's eye is located.

2. The laser system of claim 1, wherein the beam delivery arm is mounted to a device body at a position substantially posterior to the position of the shoulders of a patient when being positioned for surgery in an operational position and wherein the beam delivery arm extends to a position above the patient's eye.

3. The laser system of any one of the preceding claims, wherein the laser source is arranged to emit the laser beam in a direction away from an operator of the device.

4. The laser system of claim 3, wherein the laser beam is emitted in a direction from the head to the feet of the patient when being positioned for surgery.

5. The laser system of any one of the preceding claims, wherein the laser system is configured to be arranged adjacent to a longitudinal side of a patient bed, chair or table.

6. The laser system of claim 5, wherein the laser beam exits the beam delivery means substantially perpendicular to the plane of the bed, chair or table, the beam axis being adjustable relative to the eye of the patient when positioned for surgery.

7. The laser system of any one of the preceding claims, wherein the beam delivery arm is either fixed on the device body and/or moveable during operation of the laser.

8. The laser system of claim 7, wherein the beam delivery arm is pivotable with respect to the device body in a horizontal or other plane.

9. The laser system of any one of the preceding claims, wherein the laser device further comprises a control unit for controlling the laser source, the beam delivery arm and/or the beam directing means, and/or a display screen positioned on top of the device body and/or beam delivery means, wherein the control unit and/or the display screen are arranged at a position substantially parallel to the head of the patient when being positioned for surgery.

10. The laser system of any one of the preceding claims, wherein the laser source comprises an Excimer laser.

11. The laser system of claim 10, wherein the Excimer laser is an argon fluoride laser providing a laser beam of approximately 193 nanometers.

12. The laser system of any one of claim 1 to 11, wherein the system comprises a casing, and the ratio of the length of the beam delivery arm and the length of the casing is greater than 0.7, preferably greater than 0.9 and most preferably greater than 1.0.

13. The laser system of any one of claims 1 to 9, wherein the laser source is a solid state UV laser, and its dimensions are at most 100 cm in length, 30 cm in width and 85 cm in height.

14. The laser system of any one of the preceding claims, wherein the laser source emits a pulsed laser beam, the pulses having durations preferably of several femtoseconds to several picoseconds.
